# EUROPÄISCHE PATENTSCHRIFT

(11) **EP 2 753 299 B1**
(45) Veröffentlichungstag und Bekanntmachung des Hinweises auf die Patenterteilung: **28.09.2016**
(21) Anmeldenummer: 12745885.9
(22) Anmeldetag: 15.08.2012
(51) Int. Cl.: A61K 8/44, A61K 8/46, A61Q 11/00

(54) **SPEZIALZAHNCREME FÜR ELEKTRISCHE ZAHNBÜRSTEN**
SPECIAL TOOTHPASTE FOR ELECTRIC TOOTH BRUSHES
DENTIFRICE SPÉCIAL POUR BROSSES À DENTS ÉLECTRIQUES

(30) Priorität: 09.09.2011 DE 102011082433
(43) Veröffentlichungstag der Anmeldung: 16.07.2014
(73) Patentinhaber: Henkel AG & Co. KGaA, 40589 Düsseldorf (DE)
(72) Erfinder: ADOMAT, Christel, 40591 Düsseldorf (DE); DUSCHEK, Nicole, 40595 Düsseldorf (DE); HUNDEIKER, Claudia, 40667 Meerbusch (DE)
(86) Internationale Anmeldenummer: PCT/EP2012/065938
(87) Internationale Veröffentlichungsnummer: WO 2013/034415

(56) Entgegenhaltungen:
- WO-A2-01/62210
- WO-A2-02/26203
- US-A- 5 833 956
- US-A- 5 885 556
- US-A- 5 965 502
- US-A1- 2004 131 560
- US-A1- 2006 222 602
- US-A1- 2007 041 914
- US-B1- 6 451 291

## Beschreibung

Die Erfindung betrifft eine Zahncreme bzw. Zahnpasta, die den besonderen Erfordernissen bei der Verwendung elektrischer Zahnbürsten Rechnung trägt.

Zahnreinigungsmittel sind in verschiedenen Formen auf dem Markt und dienen in erster Linie der Reinigung der Zahnoberfläche und der Vorbeugung von Zahn- und Zahnfleischerkrankungen. Sie enthalten üblicherweise eine Kombination aus Poliermitteln, Feuchthaltemitteln, Tensiden, Bindemitteln, Aromastoffen und fluoridhaltigen sowie antimikrobiellen Wirkstoffen. Neben Zahnpulvern, die wegen ihrer erhöhten Abrasivität eine untergeordnete Rolle spielen, werden Zahnreinigungsmittel vor allem in Pasten-, Creme- und transluzenter oder transparenter Gelform angeboten. In den letzten Jahren haben auch Liquid- oder Flüssigzahncremes und Mundwässer zunehmend an Bedeutung gewonnen.

Parallel hierzu sind auch auf gerätetechnischer Seite Weiterentwicklungen verfolgt worden. Neben der herkömmlichen Handzahnbürste, die vom Verbraucher mit kreisenden Bewegungen im Mund bewegt wird, haben sich elektrische Zahnbürsten im Markt etabliert, die einen Teil der Bewegung der Borsten auf der Zahnoberfläche durch eine im Handstück befindliche - meist wiederaufladbare - Batterie erzeugen. Der Verbraucher kann hierdurch je nach Modell die kreisende Handbewegung durch eine horizontal-lineare Bewegung ersetzen.
Elektrisch bewegte Bürstenköpfe sind dabei in vielfältigen Ausgestaltungen marktüblich. So existieren runde Bürstenköpfe, die elektrisch in Rotation oder Teilrotation mit Drehungen im und gegen den Uhrzeigersinn versetzt werden. Es gibt aber auch länglich, der herkömmlichen Zahnbürste nachempfundene Bürstenköpfe, die elektrisch oszillieren.

Allen elektrischen Zahnbürsten ist der Vorteil gemein, daß sie den Verbraucher anteilsweise von bestimmten Bewegungsabläufen entlasten und im Ruf stehen, die Zähne gründlicher zu reinigen. Diesen Vorteilen stehen aber auch Nachteile gegenüber: Eine elektrische Zahnbürste führt auf der Zahnoberfläche üblicherweise zu einer intensiveren Belastung als beim Zähnputzen per Hand. Werden Art und Gehalt der Schleifkörper in der Zahncreme nicht hierauf abgestimmt, können in Verbindung mit einer elektrischen Zahnbürste Schaden am Zahnschmelz entstehen.

Ein weiteres Problem besteht darin, daß die aufwendige Mechanik im Bürstenkopf verklebt werden kann und die Zahnbürste dann die entsprechende elektrische Bewegung nicht mehr durchführen kann. Zusätzlich muß auch die Rheologie beachtet werden, denn auf den sich bewegenden Bürstenköpfen muß eine bestimmte Haftfähigkeit gegeben sein, um Verspritzen der Zahncreme zu vermeiden.

Einige der oben genannten Anforderungen stehen zueinander im Widerspruch, da Schleifkörper zumindest anteilig auch Konsistenzgeber sind.

Die WO02/26203 A1 offenbart Zahnreinigungsmittel, welche Tenside und Poliermittel enthalten. In den Beispielen werden Zusammensetzungen offenbart, bei welchen das Gewichtsverhältnis von Poliermittel(n) zu Tensid(en) 5,504 beträgt. Die Beispiele offenbaren lediglich Aniontensidgehalte von 1,5 Gew.-%, und Probleme der Bürstenkopfmechanik bei elektrischen Zahnbürsten werden nicht adressiert.

Die US 2004/0131560 A adressiert Zahnpasten, welche mit elektrischen Zahnbürsten appliziert werden können. Dieses Dokument offenbart Zusammensetzungen mit Aniontensidgehalten um die 3 Gew.-%.

Die WO2008/145475 A1, die US 2006/222602 A und die US 3 268 455 A offenbaren Zahnpasten mit Aniontensidgehalten oberhalb von 1,6 Gew.-%, adressieren aber weder das Gewichtsverhältnis von Poliermittel(n) zu Tensid(en) noch Probleme der Bürstenkopfmechanik bei elektrischen Zahnbürsten.

Die WO01/062210 A1 offenbart Zahnreinigungsmittel, welche Tenside und Poliermittel enthalten. In den Beispielen werden Zusammensetzungen offenbart, bei welchen das Gewichtsverhältnis von Poliermittel(n) zu Tensid(en) 5,504 beträgt. Die Beispiele offenbaren lediglich Aniontensidgehalte von 1,5 Gew.-%, und Probleme der Bürstenkopfmechanik bei elektrischen Zahnbürsten werden nicht adressiert.

Der vorliegenden Erfindung lag daher die Aufgabe zugrunde, ein Mund- und Zahnpflege- und -reinigungsmittel bereitzustellen, das speziell für elektrische Zahnbürsten geeignet ist und die vorstehend genannten Nachteile überwindet. Insbesondere sollte eine hohe Reinigungsleistung ohne zu hohe Abrasivität erzielt werden, verbunden mit einer Schonung der empfindlichen Mechanik der elektrisch betriebenen Bürstenköpfe und ausreichend Haftfähigkeit am Bürstenkopf.

Es wurde nun gefunden, daß anionische und amphotere und Tenside in engen Verhältnissen zueinander eingesetzt werden müssen, um die geschilderten Nachteile zu überwinden. Vorzugsweise werden auch die Einsatzmengen innerhalb enger Grenzen gewählt.

Gegenstand der vorliegenden Erfindung ist in einer ersten Ausführungsform ein Mund- und Zahnpflege- und -reinigungsmittel, enthaltend
a) mindestens ein anionisches Tensid,
b) mindestens ein amphoteres Tensid,
c) Putzkörper
wobei
- das Mittel - bezogen auf sein Gewicht- 1,6 bis 2,2 Gew.-% anionische(s) Tensid(e) enthält und
- das Gewichtsverhältnis von anionischen zu amphoteren Tensid(en) ≥ 6 ist und
- das Gewichtsverhältnis von Poliermittel(n) zu Tensid(en) im Bereich ≥ 5,25 bis ≤ 5,6 liegt.

Mund- und Zahnpflegemittel sowie Mund- und Zahnreinigungsmittel im Sinne der Erfindung sind insbesondere Mund- und Zahnpasten, flüssige Mund- und Zahncremes sowie Mund- und Zahngele. Bevorzugt geeignet sind Zahnpasten und flüssige Zahnreinigungsmittel.

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid-(ether)sulfate, Fettsäureamid(ether)sulfate, Mono- und Dialkyl-sulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Ganz besonders bevorzugte erfindungsgemäße Mittel enthalten Alkylsulfat(e) als anionisches Tensid. Erfindungsgemäß besonders bevorzugte Mund und Zahnpflege- und -reinigungsmittel sind dadurch gekennzeichnet, daß sie 1,6 bis 2,2 Gew.-% Natriumdodecylsulfat enthalten.

Als zweiten wesentlichen Inhaltsstoff enthalten die erfindungsgemäßen Mittel amphotere(s) Tensid(e). Unter ampholytischen Tensiden und Emulgatoren werden solche oberflächenaktiven Verbindungen verstanden, die außer einer C₈ - C₂₄ - Alkyl- oder -Acylgruppe mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokos-alkylaminopropionat, das Kokosacylaminoethylaminopropionat und das C₁₂ - C₁₈ - Acylsarcosin.

Besonders bevorzugte erfindungsgemäße Mund und Zahnpflege- und -reinigungsmittel sind dadurch gekennzeichnet, daß sie amphotere(s) Tensid(e) aus den Gruppen der
- N-Alkylglycine,
- N-Alkylpropionsäuren,
- N-Alkylaminobuttersäuren,
- N-Alkyliminodipropionsäuren,
- N-Hydroxyethyl-N-alkylamidopropylglycine,
- N-Alkyltaurine,
- N-Alkylsarcosine,
- 2-Alkylaminopropionsäuren mit jeweils etwa 8 bis 24 C-Atomen in der Alkylgruppe,
- Alkylaminoessigsäuren mit jeweils etwa 8 bis 24 C-Atomen in der Alkylgruppe,
- N-Kokosalkylaminopropionat,
- Kokosacylaminoethylaminopropionat
- C₁₂ - C₁₈ - Acylsarcosin,
- N-Alkyl-N,N-dimethylammonium-glycinate, beispielsweise Kokosalkyl-dimethylammoniumglycinat,
- N-Acyl-aminopropyl-N,N-dimethylammoniumglycinate, beispielsweise Kokosacylaminopropyl-dimethylammoniumglycinat,
- 2-Alkyl-3-carboxymethyl-3-hydroxyethyl-imidazoline mit jeweils 8 bis 18 C-Atomen in der Alkyl- oder Acylgruppe
- Kokosacylaminoethylhydroxyethylcarboxymethylglycinat
- der unter der INCI-Bezeichnung Cocamidopropyl Betain bekannten Verbindungen,
- der unter der INCI-Bezeichnung Disodium Cocoamphodiacetate bekannten Verbindungen
enthalten.

Besonders bevorzugte Mund und Zahnpflege- und -reinigungsmittel enthalten als amphotere Tenside Betaine der Formel (Bet-I) 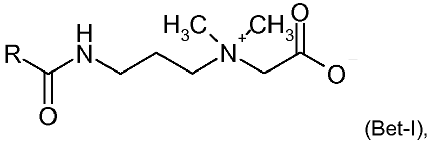 in der R für einen geradkettigen oder verzweigten, gesättigten oder ein- bzw. mehrfach ungesättigten Alkyl- oder Alkenlyrest mit 8 bis 24 Kohlenstoffatomen steht.

Diese Tenside werden nach der INCI-Nomenklatur als Amidopropylbetaine bezeichnet, wobei die Vertreter, die sich von Kokosfettsäuren ableiten, bevorzugt sind und als Cocoamidopropylbetaine bezeichnet werden. Besonders bevorzugt werden erfindungsgemäß Tenside der Formel (Bet-I) eingesetzt, die ein Gemisch der folgenden Vertreter sind:

H₃C-(CH₂)₇-C(O)-NH-(CH₂)₃N⁺(CH₃)₂CH₂COO⁻

H₃C-(CH₂)₉-C(O)-NH-(CH₂)₃N⁺(CH₃)₂CH₂COO⁻

H₃C-(CH₂)₁₁-C(0)-NH-(CH₂)₃N⁺(CH₃)₂CH₂COO⁻

H₃C-(CH₂)₁₃-C(O)-NH-(CH₂)₃N⁺(CH₃)₂CH₂COO⁻

H₃C-(CH₂)₁₅-C(O)-NH-(CH₂)₃N⁺(CH₃)₂CH₂COO⁻

H₃C-(CH₂)₇-CH=CH-(CH₂)₇-C(O)-NH-(CH₂)₃N⁺(CH₃)₂CH₂COO⁻

Besonders bevorzugt werden Tenside der Formel (Bet-I) innerhalb engerer Mengenbereiche eingesetzt.

Erfindungsgemäß beträgt das Gewichtsverhältnis von anionischen zu amphoteren Tensid(en) ≥ 6, d.h. die anionischen Tenside werden mindestens im 6-fachen (Gewichts-)Überschuß zu den amphoteren Tensiden eingesetzt. Das Gewichtsverhältnis bezieht sich dabei auf das Gewichtsverhältnis aller Stoffe aus den genannten Gruppen zueinander, wobei nur die Aktivsubstanzen gerechnet werden. Enthält ein erfindungsgemäßes Mittel z.B. 1 Gew.-% reines Natriumdodecylsulfat und 0,4 Gew.-% einer 25%igen Tensidlösung eines amphoteren Tensids, so beträgt das Gewichtsverhältnis 1 / 0,1 = 10.

Erfindungsgemäß bevorzugte Mund- und Zahnpflege- und -reinigungsmittel sind dadurch gekennzeichnet, daß das Gewichtsverhältnis von anionischen zu amphoteren Tensid(en) ≥ 6,5, vorzugsweise ≥ 6,75, weiter bevorzugt ≥ 7, noch weiter bevorzugt ≥ 7,5 besonders bevorzugt ≥ 7,75 und insbesondere ≥ 8 beträgt.

In erfindungsgemäß bevorzugten Mitteln liegt das Gewichtsverhältnis innerhalb eines noch engeren Bereiches. Besonders bevorzugte Mund und Zahnpflege- und -reinigungsmittel sind dadurch gekennzeichnet, daß das Gewichtsverhältnis von anionischen zu amphoteren Tensid(en) im Bereich ≥ 10,25 bis ≤ 20, vorzugsweise im Bereich ≥ 10,5 bis ≤ 17,5, weiter bevorzugt im Bereich ≥ 10,75 bis ≤ 15, noch weiter bevorzugt im Bereich ≥ 11 bis ≤ 12,5 und insbesondere im Bereich ≥ 11,1 bis ≤ 12 liegt.

Die erfindungsgemäßen Zusammensetzungen enthalten mindestens ein Poliermittel. Als Poliermittel eignen sich prinzipiell alle für Zahnpasten bekannten Reibkörper, insbesondere solche, die keine Calciumionen enthalten. Bevorzugt geeignete Poliermittel-komponenten sind daher Kieselsäuren, Aluminiumhydroxid, Aluminiumoxid, Natrium-aluminiumsilikate, organische Polymere oder Gemische solcher Reibkörper.

Calciumhaltige Polierkomponenten wie z.B. Kreide, Calciumpyrophosphat, Dicalcium-phosphatdihydrat können aber in Mengen bis zu 5 Gew.-% - bezogen auf die Gesamtzusammensetzung - enthalten sein.

Der Gesamtgehalt an Poliermitteln liegt vorzugsweise im Bereich von 5 - 50 Gew.-% des Zahnpflegemittels. Erfindungsgemäß bevorzugte Mittel enthalten Poliermittel innerhalb engerer Mengenbereiche. Hier sind erfindungsgemäß bevorzugte Mund- und Zahnpflege- und - reinigungsmittel dadurch gekennzeichnet, daß sie - bezogen auf ihr Gewicht - 1 bis 25 Gew.-%, vorzugsweise 2,5 bis 20 Gew.-%, weiter bevorzugt 5 bis 18 Gew.-% und insbesondere 7,5 bis 16 Gew.-% Poliermittel enthalten.

Besonders bevorzugt sind Zahnpasten und flüssige Zahnreinigungsmittel, die als Poliermittel Kieselsäuren enthalten. Geeignete Kieselsäuren sind z.B. Gelkieselsäuren, Hydrogelkieselsäuren und Fällungskieselsäuren. Gelkieselsäuren werden durch Umsetzung von Natriumsilikatlösungen mit starken, wäßrigen Mineralsäuren unter Ausbildung eines Hydrosols, Alterung zum Hydrogel, Waschen und Trocknen hergestellt. Erfolgt die Trocknung unter schonenden Bedingungen auf Wassergehalte von 15 bis 35 Gew.-%, so werden die so genannten Hydrogelkieselsäuren erhalten. Durch Trocknung auf Wassergehalte unterhalb 15 Gew.-% erfolgt eine irreversible Schrumpfung der vorher lockeren Struktur des Hydrogels zur dichten Struktur des sog. Xerogels.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Bevorzugt geeignet ist eine Fällungskieselsäure mit einer BET-Oberfläche von 15 - 110 m²/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20°C) in einer Menge von 10 - 20 Gew.-% der Zahnpaste. Bevorzugt geeignete Fällungskieselsäuren dieser Art weisen außerdem gerundete Ecken und Kanten auf und sind unter der Handelsbezeichnung Sident^{®}12 DS (DEGUSSA) erhältlich.

Andere Fällungskieselsäuren dieser Art sind Sident^{®} 8 (DEGUSSA) und Sorbosil^{®} AC 39 (Crosfield Chemicals). Diese Kieselsäuren zeichnen sich durch eine geringere Verdickungswirkung und eine etwas höhere mittlere Teilchengröße von 8 --14 µm bei einer spezifischen Oberfläche von 40 - 75 m²/g (nach BET) aus und eignen sich besonders gut für flüssige Zahncremes. Diese sollten eine Viskosität (25°C, Scherrate D = 10 s⁻¹) von 10 - 100 Pa.s aufweisen.

Zahnpasten, die eine deutlich höhere Viskosität von mehr als 100 Pa.s (25° C, D = 10 s⁻¹) aufweisen, benötigen hingegen einen genügend hohen Anteil an Kieselsäuren mit einer Teilchengröße von weniger als 5 µm, bevorzugt wenigstens 3 Gew.-% einer Kieselsäure mit einer Partikelgröße von 1 - 3 µm. Solchen Zahnpasten setzt man daher bevorzugt neben den genannten Fällungskieselsäuren noch feinteiligere, so genannte Verdickungs-Kieselsäuren mit einer BET-Oberfläche von 150 -250 m²/g zu, z.B. die Handelsprodukte Sipernat 22 LS oder Sipernat^{®} 320 DS.

Als weitere Poliermittelkomponente kann auch z.B. Aluminiumoxid in Form von schwach calcinierter Tonerde mit einem Gehalt an - und -Aluminiumoxid in einer Menge von ca. 1 - 5 Gew.-% enthalten sein. Ein solches geeignetes Aluminiumoxid ist unter der Handelsbezeichnung "Poliertonerde P10 feinst" (Giulini Chemie) erhältlich.

Als Poliermittel eignen sich weiter alle für Zahnpasten bekannten Reibkörper wie z. B. Natriumaluminiumsilikate wie z. B. Zeolith A, organische Polymere wie z. B. Polymethacrylat oder Gemische dieser und der vorstehend genannten Reibkörper.

Zusammenfassend sind erfindungsgemäße Mund- und Zahnpflege- und -reinigungsmittel bevorzugt, die zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ ·2H₂O), Natriumaluminiumsilikate, insbesondere Zeolith A, organische Polymere, insbesondere Polymethacrylate oder Gemische dieser Reibkörper, vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthalten.

Erfindungsgemäß besonders bevorzugte Mund und Zahnpflege- und -reinigungsmittel enthalten ausschließlich Poliermittel aus der Gruppe der Kieselsäuren, Aluminiumhydroxid, Aluminiumoxid, Dicalciumphosphat-dihydrat (CaHPO₄ ·2H₂O) oder Gemische dieser Reibkörper. Diese Poliermittel haben ich als besonders effizient bei der Lösung der erfindungsgemäßen Aufgabe erwiesen.

Ganz besonders bevorzugt enthalten die erfindungsgemäßen Zusammensetzungen bezogen auf ihr Gewicht 1 bis 30 Gew.-% Fällungskieselsäure(n) mit einer spezifischen Oberfläche nach ISO 5794-1, Anhang D von ≤ 55 m²/g. Vorzugsweise werden die Fällungskieselsäuren, die entsprechende spezifische Oberflächen aufweisen, innerhalb engerer Mengenbereiche eingesetzt, und insbesondere bevorzugt werden Fällungskieselsäuren eingesetzt, die noch niedrigere spezifische Oberflächen nach ISO 5794-1, Anhang D aufweisen. Bevorzugte erfindungsgemäße Mund- und Zahnpflege- und -reinigungsmittel enthalten 2,5 bis 25 Gew.-%, vorzugsweise 5 bis 20 Gew.-%, besonders bevorzugt 7,5 bis 17,5 Gew.-%, weiter bevorzugt 8,0 bis 15,0 Gew.-% und insbesondere 10,0 bis 13,0 Gew.-% Fällungskieselsäure(n) mit einer spezifischen Oberfläche nach ISO 5794-1, Anhang D von ≤ 55 m²/g.

Besonders bevorzugte erfinddungsgemäße Mund und Zahnpflege- und -reinigungsmittel sind dadurch gekennzeichnet, daß sämtliche im Mittel enthaltenen Fällungskieselsäure(n) eine spezifische Oberfläche nach ISO 5794-1, Anhang D von ≤ 53 m²/g, vorzugsweise von ≤ 51 m²/g, weiter bevorzugt von ≤ 49 m²/g und insbesondere von ≤ 47 m²/g aufweisen.

In weiter bevorzugten erfindungsgemäßen Mitteln sind die eingesetzten Fällungskieselsäuren durch weitere physikalische Parameter gekennzeichnet. Vorzugsweise einzusetzende Fällungskieselsäuren weisen Stampfdichten > 360 g/l (gemessen nach ISO 787-11), besonders bevorzugt > 375 g/l, weiter bevorzugt > 400 g/l und insbesondere > 425 g/l auf.

Es ist weiter bevorzugt, Fällungskieselsäuren einzusetzen, die eine DBP-Absorption gemäß DIN 53601 von weniger als 140 g/100 g aufweisen. Ganz besonders bevorzugte erfindungsgemäß einzusetzende Fällungskieselsäuren weisen eine DBP-Absorption gemäß DIN 53601 von weniger als 135 g/100 g, bevorzugt von eine DBP-Absorption gemäß DIN 53601 von weniger als 130 g/100 g und insbesondere von weniger als 125 g/100 g auf.

Erfindungsgemäß besonders bevorzugte Mittel enthalten 2,5 bis 25 Gew.-%, vorzugsweise 5 bis 20 Gew.-%, besonders bevorzugt 7,5 bis 17,5 Gew.-%, weiter bevorzugt 8,0 bis 15,0 Gew.-% und insbesondere 10,0 bis 14,0 Gew.-% Fällungskieselsäure(n) mit einer spezifischen Oberfläche nach ISO 5794-1, Anhang D von ≤ 45 m²/g und einer Stampfdichte (gemessen nach ISO 787-11), von > 425 g/l.

Erfindungsgemäß weiter bevorzugte Mittel enthalten 2,5 bis 25 Gew.-%, vorzugsweise 5 bis 20 Gew.-%, besonders bevorzugt 7,5 bis 17,5 Gew.-%, weiter bevorzugt 8,0 bis 15,0 Gew.-% und insbesondere 10,0 bis 14,0 Gew.-% Fällungskieselsäure(n) mit einer spezifischen Oberfläche nach ISO 5794-1, Anhang D von ≤ 45 m²/g und einer DBP-Absorption gemäß DIN 53601 von weniger als 125 g/100 g.

Erfindungsgemäß insbesondere bevorzugte Mittel enthalten 2,5 bis 25 Gew.-%, vorzugsweise 5 bis 20 Gew.-%, besonders bevorzugt 7,5 bis 17,5 Gew.-%, weiter bevorzugt 8,0 bis 15,0 Gew.-% und insbesondere 10,0 bis 14,0 Gew.-% Fällungskieselsäure(n) mit einer spezifischen Oberfläche nach ISO 5794-1, Anhang D von ≤ 45 m²/g und einer Stampfdichte (gemessen nach ISO 787-11), von > 425 g/l und einer DBP-Absorption gemäß DIN 53601 von weniger als 125 g/100 g.

Es hat sich gezeigt, daß die Einsatzmenge von Poliermittel(n) an die Gesamtmenge an Tensid(en) angepaßt wird. Im Hinblick auf die erfindungsgemäße Aufgabenstellung sind Mittel, bei denen das Gewichtsverhältnis von Poliermittel(n) zu Tensid(en) im Bereich ≥ 5,25 bis ≤ 5,6 liegt, gekennzeichnet. Das Gewichtsverhältnis bezieht sich dabei auf das Gewichtsverhältnis aller Stoffe aus den genannten Gruppen zueinander, wobei nur die Aktivsubstanzen gerechnet werden. Enthält ein erfindungsgemäßes Mittel z.B. 12 Gew.-% Poliermittel und 4 Gew.-% einer 50%igen Tensidlösung, so beträgt das Gewichtsverhältnis 12/2=6. Enthält ein erfindungsgemäßes Mittel z.B. 12 Gew.-% Poliermittel und 4 Gew.-% einer 50%igen Tensidlösung sowie 1 Gew.-% reines Natriumdodecylsulfat, so beträgt das Gewichtsverhältnis 12 / (2 + 1) = 4.

Mund- und Zahnpflege- und reinigungsmittel, insbesondere Zahnpasten, können z.B. auch Substanzen enthalten, die gegen Plaque und/oder Zahnstein wirksam sind.

Zahnbelag (Plaque) ist ein rauer, klebriger Belag auf den Zähnen, der aus Speichel, Bakterien und Nahrungsresten besteht. Setzen sich Mineralsalze (z.B. Calcium, Phosphat) aus dem Speichel im Zahnbelag ab, so bilden sich harte, weiße oder gelbliche Ablagerungen am Zahn, die man Zahnstein nennt. In dem porösen Zahnstein kann sich wiederum leicht Zahnbelag absetzen, der das Zahnfleisch angreift.

Die Bakterien auf der Zahnoberfläche bauen Kohlenhydrate, besonders Zucker, aus der Nahrung zu Säure ab. Diese Säure löst die Zahnsubstanz auf und es kommt zu Karies (Zahnfäule). Dabei werden besonders die Mineralien Calcium und Phosphat aus dem Zahnschmelz herausgelöst. Nach dem Zahnschmelzmantel werden auch innere Schichten des Zahnes angegriffen. Bakterien können in das Zahnmark eindringen und dort zu Entzündungen führen. Meist kommt es dann zu stechenden Zahnschmerzen.

Wie bereits erwähnt, beinhaltet Plaque Bakterien, so daß sich zur Bekämpfung von Plaque antimikrobielle Stoffe eignen. Diese besitzen darüber hinaus eine Wirkung als Konservierungsmittel.

In Mund und Zahnpflege- und -reinigungsmitteln können beispielsweise die auch in Lebensmitteln zugelassenen Konservierungsmittel Sorbinsäure (E 200), Kaliumsorbat (E 202), Calciumsorbat (E 203), Benzoesäure (E 210), Natriumbenzoat (E 211), Kaliumbenzoat (E 212), Calciumbenzoat (E 213), Ethyl-4-hydroxybenzoat (E 214), Ethyl-4-hydroxybenzoat, Natriumsalz (E 215), Propyl-4-hydroxybenzoat (E 216), Propyl-4-hydroxybenzoat, Natriumsalz (E 217), Methyl-4-hydroxybenzoat (E 218), Methyl-4-hydroxybenzoat, Natriumsalz (E 219), Schwefeldioxid (schweflige Säure), (E 220), Natriumsulfit (E 221), Natriumhydrogensulfit (E 222), Natriumdisulfit (E 223), Kaliumdisulfit (E 224), Calciumsulfit (E 226), Calciumhydrogensulfit (E 227), Kaliumhydrogensulfit (E 228), Biphenyl (E 230), Orthophenylphenol (2-Biphenylol), (E 231), Natriumorthophenylphenolat (E 232), Nisin (E 234), Natamycin (E 235), Ameisensäure (E 236), Natriumformiat (E 237), Calciumformiat (E 238), Hexamethylentetramin (E 239), Dimethyldicarbonat (E 242), Kaliumnitrit (E 249), Natriumnitrit (E 250), Natriumnitrat (E 251), Essigsäure (E 260), Kaliumacetat (E 261), Natriumacetat (E 262), Calciumacetat (E 263), Milchsäure (E 270), Propionsäure (E 280), Natriumpropionat (E 281), Calciumpropionat (E 282), Kaliumpropionat (E 283), Borsäure (E 284), Natriumtetraborat (E 285), Hydroxybernsteinsäure (Äpfelsäure), (E 296), Fumarsäure (E 297), Lysozym (E 1105), eingesetzt werden.

Bevorzugte Stoffe sind ausgewählt aus Alkypyridiniumsalzen, insbesondere Cetylpyridiniumchlorid, p-Hydroxybenzoesäuremethyl-, -ethyl- oder propylester, Natriumsorbat, Natriumbenzoat, Bromchlorophen, Triclosan, Phenyl- Salicylsäureester, Biguaniden z. B. Chlorhexidin (1,1'-Hexamethylenbis[5-(4-chlorphenyl)-biguanid), Thymol usw..

Erfindungsgemäß bevorzugte Mund- und Zahnpflege- und -reinigungsmittel sind dadurch gekennzeichnet, daß sie zusätzlich Antiplaque-Wirkstoffe, vorzugsweise Alkypyridiniumsalze, insbesondere Cetylpyridiniumchlorid, p-Hydroxybenzoesäuremethyl-, -ethyl- oder propylester, Natriumsorbat, Natriumbenzoat, Bromchlorophen, Triclosan, Hexetidine, Phenyl- Salicylsäureester, Biguanide z. B. Chlorhexidin, Thymol, vorzugsweise in Mengen von 0,05 bis 5 Gew.%, vorzugsweise von 0,10 bis 2,5 Gew.% und insbesondere von 0,30 bis 1,5 Gew.%, jeweils bezogen auf das gesamte Mittel, enthalten.

Die angegebenen Mengen beziehen sich auf die Gesamtmenge an Antiplaque-Wirkstoffen, wobei einzelne Wirkstoffe vorzugsweise in engeren Mengenbereichen eingesetzt werden. Erfindungsgemäß bevorzugte Mittel enthalten beispielsweise 0,1 bis 2 Gew.-%, vorzugsweise 0,2 bis 1,75 Gew.-%, weiter bevorzugt 0,3 bis 1,5 Gew.-% und insbesondere 0,4 bis 1,0 Gew.-% Natriumbenzoat. Weiter bevorzugte erfindungsgemäße Mittel sind dadurch gekennzeichnet, daß sie 0,005 bis 0,1 Gew.-%, vorzugsweise 0,01 bis 0,075 Gew.-% und insbesondere 0,02 bis 0,05 Gew.-% Chlorhexidin enthalten. Chlorhexidin wird vorzugsweise gemeinsam mit Alkylpolyglycosiden (APG) eingesetzt, wobei in bevorzugten erfindungsgemäßen Mitteln als Alkylglykoside solche mit 8-18 C-Atomen in der Alkylgruppe und einem mittleren Oligomerisationsgrad des Glycosidrestes von 1-3 in einer Menge von 0,025 bis 2,5 Gew.-% enthalten sind.

Gegen Zahnstein wirksame Stoffe können beispielsweise Chelatbildner sein wie z. B. Ethylendiamintetraessigsäure und deren Natriumsalze, Pyrophosphat- Salze wie die wasserlöslichen Dialkali- oder Tetraalkalimetallpyrophosphat- Salze, z. B. Na₄P₂O₇, K₄P ₂O₇, Na₂K₂P₂O₇, Na₂H₂P₂O₇ und K₂H₂P₂O₇ oder Polyphosphat-Salze, die z. B. aus wasserlöslichen Alkalimethalltripolyphosphaten wie Natriumtripolyphosphat und Kaliumtripolyphosphat ausgewählt sein können.

Erfindungsgemäß bevorzugte Mund- und Zahnpflege- und -reinigungsmittel sind dadurch gekennzeichnet, daß sie zusätzlich Phosphat(e), vorzugsweise Alkalimetallphosphat(e) und insbesondere Natriumtripolyphosphat, vorzugsweise in Mengen von 1 bis 10 Gew.-%, besonders bevorzugt von 2 bis 8 Gew.-% und insbesondere von 3 bis 7 Gew.-%, jeweils bezogen auf das gesamte Mittel, enthalten.

Als Konsistenzregler (bzw. Bindemittel) dienen z. B. natürliche und/oder synthetische wasserlösliche Polymere wie Alginate, Carragheenate, Traganth, Stärke und Stärkeether, Celluloseether wie z. B. Carboxymethylcellulose (Na-Salz), Hydroxyethylcellulose, Methylhydroxypropylcellulose, Guar, Akaziengum, Agar-Agar, Xanthan- Gum, Succinoglycan-Gum, Johannisbrotmehl, Pectine, wasserlösliche Carboxyvinylpolymere (z. B. Carbopol®-Typen), Polyvinylalkohol, Polyvinylpyrrolidon, Polyethylenglycole, insbesondere solche mit Molekulargewichten von 1 500-1 000 000.

Weitere Stoffe, die sich zur Viskositätskontrolle eignen, sind z. B. Schichtsilikate wie z. B. Montmorillonit-Tone, kolloidale Verdickungskieselsäuren wie z. B. Aerogel-Kieselsäuren, pyrogene Kieselsäuren oder feinstvermahlene Fällungskieselsäuren. Es können auch viskositätsstabilisierende Zusätze aus der Gruppe der kationischen, zwitterionischen oder ampholytischen stickstoffhaltigen Tenside, der hydroxypropylsubstituierten Hydrocolloide oder der Polyethylenglycol/Polypropylenglycol- Copolymere mit einem mittleren Molgewicht von 1000 bis 5000 oder eine Kombination der genannten Verbindungen in den Zahnpasten verwendet werden.

Die erfindungsgemäßen Mittel, insbesondere die Zahnpasten, können auch Substanzen zur Erhöhung des mineralisierenden Potentials enthalten, beispielsweise calciumhaltige Substanzen wie z. B. Calciumchlorid, Calciumacetat und Dicalciumphosphat-Dihydrat. Die Konzentration der calciumhaltigen Substanz hängt von der Löslichkeit der Substanz und dem Zusammenwirken mit anderen in dem Mund- und Zahnpflegemittel enthaltenen Substanzen ab.

Neben den genannten obligatorischen Komponenten können die erfindungsgemäßen Zahnpflegemittel weitere, an sich bekannte Hilfs- und Zusatzstoffe enthalten. Dabei ist ein Zusatzstoff, der als Zahnpastenkomponente seit langem bekannt ist, in den erfindungsgemäßen Zahnpflegemitteln besonders wirksam: Calcium-glycerophosphat, das Calcium-Salz der Glycerin-1-phosphorsäure oder der Glycerin-2-phosphorsäure oder der zur Glycerin-1-phosphorsäure enantiomeren Glycerin-3-phosphorsäure - oder eines Gemisches dieser Säuren. Die Verbindung hat in Zahnpflegemitteln eine remineralisierende Wirkung, da sie sowohl Calcium- als auch Phosphationen liefert. In den erfindungsgemäßen Zahnpflegemitteln wird Calciumglycerophosphat bevorzugt in Mengen von 0,01 - 1 Gew.-% eingesetzt. Insgesamt können die erfindungsgemäßen Zahnreinigungsmittel übliche Hilfsmittel und Zusatzstoffe in Mengen bis zu 10 Gew.-% enthalten.

Mund und Zahnpflege- und -reinigungsmittel können darüber hinaus mit besonderem Vorzug Antikaries-Wirkstoffe enthalten. Diese können beispielsweise aus organischen oder anorganischen Fluoriden ausgewählt sein, z. B. aus Natriumfluorid, Kaliumfluorid, Natriummonofluorphosphat und Natriumfluorosilikat. Auch Zinkfluorid, Zinn-(II)-fluorid sind bevorzugt. Bevorzugt sollte eine Menge von 0,01 - 0,2 Gew.-% Fluor in Form der genannten Verbindungen enthalten sein.

Erfindungsgemäße Mund- und Zahnpflege- und -reinigungsmittel, die zusätzlich Antikaries-Wirkstoffe, vorzugsweise Fluorverbindung(en), insbesondere Natriumfluorid, Kaliumfluorid, Natriummonofluorphosphat, Zinkfluorid, Zinnfluorid und Natriumfluorosilikat, vorzugsweise in Mengen von 0,01 bis 5 Gew.%, vorzugsweise von 0,1 bis 2,5 Gew.% und insbesondere von 0,2 bis 1,1 Gew.%, jeweils bezogen auf das gesamte Mittel, enthalten, sind erfindungsgemäß bevorzugt.

Die erfindungsgemäßen Zahnpflegemittel können z.B. durch Zusatz von Aromaölen und Süßungsmitteln in ihren organoleptischen Eigenschaften verbessert werden.

Als Aromaöle können alle die für Mund- und Zahnpflegemittel üblichen natürlichen und synthetischen Aromen eingesetzt werden. Natürliche Aromen können sowohl in Form der aus Drogen isolierten natürlichen ätherischen Öle als auch der daraus isolierten Einzelkomponenten enthalten sein.

Geeignete Aromen sind z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser Komponenten.

Geeignete Süßungsmittel sind z.B. Saccharin-Natrium, Natrium-Cyclamat, Sucrose, Lactose, Meltose, Fructose sowie Maltose und Dextrose.

### Beispiele:

Folgende Zahncremes wurden hergestellt und unter gleichen Bedingungen auf einer elektrischen Zahnbürste verwendet.

**Angaben in %**

| | **V1** | **V2** | **E** |
|---|---|---|---|
| Sorbitol 70% | 18,5 | 18,5 | 18,5 |
| Glycerin | 22,5 | 22,5 | 22,5 |
| Kieselsäure abrasiv | 12 | 12 | 12 |
| Kieselsäure verdickend | 9 | 9 | 9 |
| Cl 42090 | 0,005 | 0,005 | 0,005 |
| Kieselsäurepartikel | 1,5 | 1,5 | 1,5 |
| Dinatriumphosphat | 0,2 | 0,2 | 0,2 |
| Trinatriumphosphat | 0,25 | 0,25 | 0,3 |
| Natriumfluorid | 0,32 | 0,32 | 0,32 |
| Saccharin | 0,25 | 0,25 | 0,25 |
| Zinksulfat 7H2O | 0,09 | 0,09 | 0,09 |
| Xanthan FN | 0,9 | 0,9 | 0,9 |
| Cocamidopropylbetain | 0 | 1 | 0,2 |
| Natriumlaurylsulfat | 2,2 | 1,2 | 2 |
| Aroma | 1 | 1 | 1 |
| Wasser | add 100 | add 100 | add 100 |

Rezeptur V1 enthielt kein amphoteres Tensid, in Rezeptur V2 betrug das Gewichtsverhältnis von anionischen zu amphoteren Tensid(en) 1,2 und nur in Rezeptur E betrug das Gewichtsverhältnis von anionischen zu amphoteren Tensid(en) 10 (entsprechend ≥ 8).

Die Zusammensetzungen wurden mittels einer elektrischen Zahnbürste appliziert.

Parameter für die Anwendung auf elektrischer Zahnbürste:
- 1g Zahncreme
- Putzzeit 3min
- Putzuntergrund: wellenförmige Platte

Anschließend wurden Fotoaufnahmen der Bürstenköpfe angefertigt.

Zudem wiesen die Bürstenköpfe der elektrischen Zahnbürste nach jeweils 100 Durchgängen (Abspülen unter fließendem Wasser zwischen den Durchgängen) bei den Zusammensetzungen V1 und V2 erhebliche Verkrustungen auf. Nach 200 Durchgängen war die Mechanik in den Bürstenköpfen der elektrischen Zahnbürste bei den Zusammensetzungen V1 und V2 stark beeinträchtigt, während sie im Falle der Zusammensetzung E problemlos funktionierte.

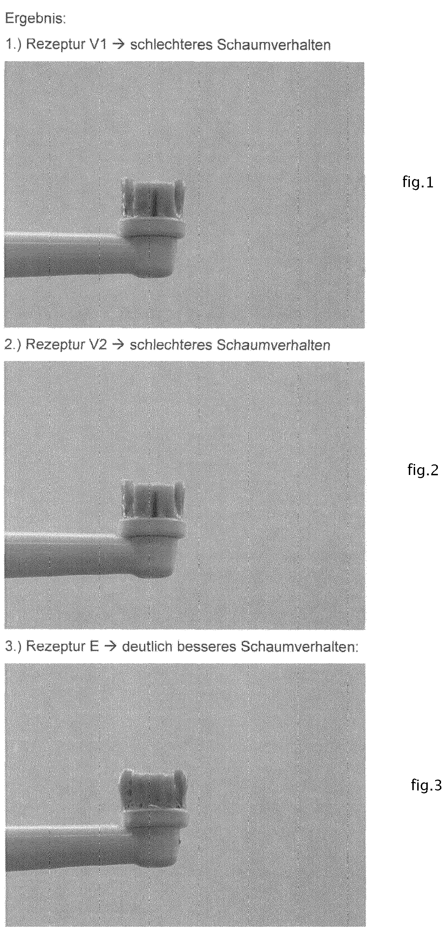

## Patentansprüche

1. Mund- und Zahnpflege- und -reinigungsmittel, enthaltend
a) mindestens ein anionisches Tensid,
b) mindestens ein amphoteres Tensid,
c) Putzkörper
**dadurch gekennzeichnet, dass**
- es - bezogen auf sein Gewicht - 1,6 bis 2,2 Gew.-% anionische(s) Tensid(e) enthält und
- das Gewichtsverhältnis von anionischen zu amphoteren Tensid(en) ≥ 6 ist und
- das Gewichtsverhältnis von Poliermittel(n) zu Tensid(en) im Bereich ≥ 5,25 bis ≤ 5,6 liegt.

2. Mund- und Zahnpflege- und -reinigungsmittel nach Anspruch 1, **dadurch gekennzeichnet, dass** das Gewichtsverhältnis von anionischen zu amphoteren Tensid(en) ≥ 6,5, vorzugsweise ≥ 6,75, weiter bevorzugt ≥ 7, noch weiter bevorzugt ≥ 7,5 besonders bevorzugt ≥ 7,75 und insbesondere ≥ 8 ist.

3. Mund und Zahnpflege- und -reinigungsmittel nach einem der Ansprüche 1 oder 2, **dadurch gekennzeichnet, dass** das Gewichtsverhältnis von anionischen zu amphoteren Tensid(en) im Bereich ≥ 10,25 bis ≤ 20, vorzugsweise im Bereich ≥ 10,5 bis ≤ 17,5, weiter bevorzugt im Bereich ≥ 10,75 bis ≤ 15, noch weiter bevorzugt im Bereich ≥ 11 bis ≤ 12,5 und insbesondere im Bereich ≥ 11,1 bis ≤ 12 liegt.

4. "Mund- und Zahnpflege- und -reinigungsmittel nach einem der Ansprüche 1 bis 3, **dadurch gekennzeichnet, dass** es als Putzkörper Kieselsäuren, Aluminiumhydroxid, Aluminiumoxid, Calciumpyrophosphat, Kreide, Dicalciumphosphat-dihydrat (CaHPO₄ ·2H₂O), Natriumaluminiumsilikate, insbesondere Zeolith A, organische Polymere, insbesondere Polymethacrylate oder Gemische dieser Reibkörper, vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthält.

## Claims

1. An oral and dental care and cleaning agent, containing
a) at least one anionic surfactant,
b) at least one amphoteric surfactant,
c) cleaning particles,
**characterized in that**
- said agent contains - based on its weight - 1.6 to 2.2 wt.% anionic surfactant(s), and
- the weight ratio of anionic to amphoteric surfactant(s) is ≥ 6, and
- the weight ratio of polishing agent(s) to surfactant(s) is in the range of from ≥ 5.25 to ≤ 5.6.

2. The oral and dental care and cleaning agent according to claim 1, **characterized in that** the weight ratio of anionic to amphoteric surfactant(s) is ≥ 6.5, preferably ≥ 6.75, more preferably ≥ 7, even more preferably ≥ 7.5, most preferably ≥ 7.75 and in particular ≥ 8.

3. The oral and dental care and cleaning agent according to either claim 1 or claim 2, **characterized in that** the weight ratio of anionic to amphoteric surfactant(s) is in the range of from ≥ 10.25 to ≤ 20, preferably in the range of from ≥ 10.5 to ≤ 17.5, more preferably in the range of from ≥ 10.75 to ≤ 15, even more preferably in the range of from ≥ 11 to ≤ 12.5, and in particular in the range of from ≥ 11.1 to ≤ 12.

4. The oral and dental care and cleaning agent according to one of claims 1 to 3, **characterized in that** it comprises as cleaning particles silicic acids, aluminum hydroxide, aluminum oxide, calcium pyrophosphate, chalk, dicalcium phosphate dihydrate (CaHPO₄ 2H₂O), sodium aluminosilicates, in particular zeolite A, organic polymers, in particular polymethacrylates, or mixtures of these abrasives, preferably in amounts of from 1 to 30 wt.%, preferably of from 2.5 to 25 wt.%, and in particular of from 5 to 22 wt.%, in each case based on the total agent.

## Revendications

1. Agent de nettoyage et de soin bucco-dentaires, contenant
a) au moins un tensioactif anionique,
b) au moins un tensioactif amphotère,
c) un abrasif
**caractérisé en ce que**
- il contient sur la base de son poids 1,6 à 2,2% en poids du ou des tensio-actifs anioniques, et
- le rapport en poids du ou des tensioactifs anioniques sur le ou les tensioactifs amphotères est ≥ 6, et
- le rapport en poids du ou des agents de polissage sur le ou les tensioactifs est dans la gamme ≥ 5,25 à ≤ 5,6.

2. Agent de nettoyage ou de soins bucco-dentaires selon la revendication 1, **caractérisé en ce que** le rapport en poids du ou des tensioactifs anioniques sur le ou les tensioactifs amphotères ≥ 6,5, de préférence ≥ 6,75, plus préférablement ≥ 7, encore plus préférablement ≥ 7,5, de manière particulièrement préférée ≥ 7,75 et en particulier ≥ 8.

3. Agent de nettoyage et de soins bucco-dentaires selon l'une des revendications 1 ou 2, **caractérisé en ce que** le rapport en poids du ou des tensioactifs anioniques sur le ou les tensioactifs amphotères est dans la gamme de ≥ 10,25 à ≤ 20, de préférence dans la gamme de ≥ 10,5 à ≤ 17,5, plus préférablement dans la gamme de ≥ 10,75 à ≤ 15, encore plus préférablement dans la gamme ≥ 11 à ≤ 12,5 et en particulier dans la gamme ≥ 11,1 à ≤ 12.

4. Agent de nettoyage et de soins bucco-dentaires selon l'une des revendications 1 à 3, **caractérisé en ce qu'**il contient comme abrasif des acides siliciques, de l'hydroxyde d'aluminium, de l'oxyde d'aluminium, du pyrophosphate de calcium, de la craie, du phosphate dicalcique dihydraté (CaHPO₄·2H₂O), des silicates d'aluminium et de sodium, en particulier de la zéolithe A, des polymères organiques, en particulier des polyméthacrylates ou des mélanges de ces abrasifs, de préférence dans des quantités de 1 à 30% en poids, de préférence de 2,5 à 25% en poids et en particulier de 5 à 22% en poids, à chaque fois par rapport à tout l'agent.
